# EUROPÄISCHE PATENTSCHRIFT

(11) **EP 3 873 372 B1**
(45) Veröffentlichungstag und Bekanntmachung des Hinweises auf die Patenterteilung: **24.07.2024**
(21) Anmeldenummer: 19798015.4
(22) Anmeldetag: 28.10.2019
(51) Int. Cl.: A61B 50/20, A61B 90/70, A61C 19/00, A61B 1/12

(54) **HALTEVORRICHTUNG UND PRODUKTÜBERWACHUNGSSYSTEM**
HOLDING APPARATUS AND PRODUCT MONITORING SYSTEM
DISPOSITIF DE RETENUE ET SYSTÈME DE SURVEILLANCE DE PRODUIT

(30) Priorität: 29.10.2018 DE 102018126969
(43) Veröffentlichungstag der Anmeldung: 08.09.2021
(73) Patentinhaber: Aesculap AG, 78532 Tuttlingen (DE)
(72) Erfinder: HÖGERLE, Roland-Alois, 78532 Tuttlingen (DE); AUBER, Stephanie, 78532 Tuttlingen (DE)
(74) Vertreter: Winter, Brandl - Partnerschaft mbB
(86) Internationale Anmeldenummer: PCT/EP2019/079342
(87) Internationale Veröffentlichungsnummer: WO 2020/089142

(56) Entgegenhaltungen:
- EP-A1- 2 514 386
- WO-A1-03/092524
- WO-A1-2008/020770
- DE-A1- 102009 051 619
- DE-A1- 102015 108 264
- JP-A- 2009 136 492
- JP-A- 2009 172 013
- US-A1- 2009 261 549

## Beschreibung

Die vorliegende Erfindung betrifft eine Haltevorrichtung zum Halten zumindest eines sterilisierbaren Medizinprodukts, insbesondere während eines Reinigungsvorgangs sowie zum Lagern des Medizinprodukts vor und/ oder nach dem Reinigungsvorgang.

### Hintergrund der Erfindung

Haltevorrichtungen der vorliegenden Gattung werden unter anderem zur Reinigung und Halterung von Motoren und Handstücken chirurgischer Instrumente bereitgestellt und sind unter anderem in der DE 10 2010 017 624 beschrieben. Die Halterungen werden mit in den Halterungen angeordneten Medizinprodukten in medizinischen Reinigungsanlagen, insbesondere Sterilisatoren, Spülanlangen oder Ähnlichen, angeordnet. Darüber hinaus besteht auch die Möglichkeit, dass die Halterungen zusammen mit Sterilcontainern verwendet und innerhalb dieser angeordnet werden können, sodass nach einer Sterilisation der Medizinprodukte gewährleistet ist, dass eine neuerliche Kontamination der Medizinprodukte wirksam unterbunden ist.

Es ist darüber hinaus auch üblich, dass beispielsweise wiederverwendbare Implantate in ähnlicher Weise wiederaufbereitet werden, wie die erwähnten chirurgischen Instrumente. Bei einer Vielzahl dieser wiederaufbereitbaren Implantate, insbesondere wenn sie aus Titan gefertigt sind, führt eine häufige Wiederaufbereitung zu Qualitätsverlusten bei dem Medizinprodukt. Bei Titanschrauben ist es beispielsweise so, dass sich die Dicke, der die Titanschraube vor Korrosion und Biofilmbildung schützenden Oxidschicht, durch die häufige Verwendung aggressiver Reinigungssubstanzen verringert. Wiederaufbereitbare Implantate liegen häufig in Sets vor, die, unabhängig davon ob einzelne Elemente des Sets tatsächlich eingesetzt werden, stets gemeinsam einen Aufbereitungsprozess durchlaufen.

### Stand der Technik

Aus dem Stand der Technik, beispielsweise gemäß einschlägiger Produkte der vorliegenden Anmelderin selbst, sind Halterungen der vorstehend beschriebenen Gattung bekannt, die die darin gehaltenen Medizinprodukte über den gesamten Reinigung- und Aufbereitungsprozess begleiten. Eine derartige Halterung ist beispielsweise aus der DE 10 2010 017 624 bekannt.

Um nachvollziehen zu können wie oft ein Medizinprodukt während seiner Lebensdauer wiederaufbereitet worden ist, ist es beispielsweise bekannt, in einem Motor des Medizinprodukts integrierte Temperatursensoren mit eigenständigen Energieversorgungseinheiten oder Energyharvestingeinheiten vorzusehen, die die Temperatur während des Reinigungsvorgangs erfassen und in einem Datenspeicher des Motors speichern. Ein Motor mit hierzu geeigneten Speichereinrichtungen ist beispielsweise in der DE 10 2011 050 192 beschrieben. Hierbei ist insbesondere nachteilig, dass eine derartige Lösung einen verhältnismäßig großen Bauraum in dem Medizinprodukt einnimmt und dessen Gewicht erhöht. Insbesondere bei Medizinprodukten zur Anwendung im Bereich der Neurochirurgie, bei minimalinvasiven Verfahren, oder Ähnlichem sind diese Nachteile nicht akzeptabel.

Als wichtig hat sich bei der Überwachung von Medizinprodukten erwiesen, dass
- eine Veränderung von Volumen und/ oder Gewicht des Medizinprodukts selbst möglichst vermieden ist und
- eine Überwachung einzelner Medizinprodukte ermöglicht wird.

Darüber hinaus wäre es wünschenswert, dass
- die erfassten Daten drahtlos ausgelesen werden können,
- die erfassten Daten einer Auswertung zugeführt werden können und
- auf die Daten weltweit zugegriffen werden kann.

Aus der EP 2 514 386 A1 ist ein medizinischer Adapter zum Nachweis einer Reinigung bzw. Pflege eines medizinischen Instruments bekannt. Der Adapter weist einen Anschluss für das medizinische Instrument und einen Anschluss für eine Fluidquelle auf.

Die US 2009/261549 A1 offenbart einen Wagen mit Aufnahmen für Behälter, wobei Instrumente in den Behältern gereinigt werden. Behälter betreffende Daten können an ein zentrales Informationssystem kommuniziert werden. Ein Näherungssensor erfasst, ob sich ein Behälter in einer Aufnahme des Wagens befindet.

Die WO 2008/020770 A1 offenbart ein Sterilisationsgerät mit einer Bodenwanne und einem Deckel. Eine Abflusssteuerungsvorrichtung weist einen Füllstandssensor auf, welcher mit einer Datenverarbeitungseinrichtung verbunden ist, so dass einem Nutzer über ein Display angezeigt werden kann, dass ein erforderlicher Sterilisierflüssigkeitsbetrag in den Aufnahmeraum eingebracht wurde. In das Sterilisationsgerät kann die zu sterilisierende Ausrüstung gelegt werden. Die Abflusssteuerungsvorrichtung nimmt dabei Datum, Zeit und Zyklusanzahl auf.

Die DE 10 2009 051619 A1 offenbart eine Pflegevorrichtung mit einer Reinigungskammer, in welcher dentaltechnische Instrumente auf Anschlussstutzen aufgesteckt werden können. Es sind Drucksensoren vorgesehen, über welche ein zur Verfügung stehendes Medienvolumen in den Vorratsgefäßen ermittelt werden kann. Zur Steuerung der Prozesse ist eine Auswerteeinheit vorgesehen.

Die JP 2009 172013 A und die JP 2009 136492 A offenbaren jeweils eine Wasch-/ bzw. Desinfektionsvorrichtung.

Die WO 03/092524 A1 offenbart eine Haltevorrichtung gemäß der Präambel des Anspruchs 1.

Die DE 10 2015 108264 A1 offenbart ein Behälterinhalt-Erfassungssystem, welches einer Identifikation von Medizinprodukten in einem Sterilisationsbehälter dient.

### Kurzbeschreibung der Erfindung

Angesichts vorstehender Beschreibung des Stands der Technik ist es die Aufgabe der vorliegenden Erfindung, eine Haltevorrichtung für sterilisierbare Medizinprodukte bereitzustellen, mittels derer die vorstehend genannten wünschenswerten Eigenschaften möglichst sämtlich erreichbar sind.

Diese Aufgabe wird durch eine Haltevorrichtung mit den Merkmalen des Patentanspruchs 1 bzw. durch ein Produktüberwachungssystem mit einer solchen Haltevorrichtung gelöst. Vorteilhafte Ausgestaltungen/Weiterbildungen der Erfindung sind Gegenstand der Unteransprüche.

Die vorliegende Erfindung betrifft demzufolge eine Haltevorrichtung zur Verwendung innerhalb eines zu reinigenden und innerhalb eines zu sterilisierenden Bereichs, zum Halten zumindest eines sterilisierbaren Medizinprodukts während eines Reinigungsvorgangs sowie zum Lagern des Medizinprodukts vor und nach dem Reinigungsvorgang. Erfindungsgemäß weist die Haltevorrichtung eine Datenverarbeitungseinrichtung auf, die mit zumindest einem Temperatursensor zur Bestimmung einer Temperatur während des Reinigungsvorgangs signalleitend verbunden/ verbindbar ist. Der Temperatursensor ist eingerichtet, einen Temperaturverlauf bzw. eine Temperaturkurve zu erfassen und an die Datenverarbeitungseinrichtung weiterzugeben. Die Datenverarbeitungseinrichtung verarbeitet die Sensorsignale zu Reinigungsinformationen und weist eine Speichervorrichtung auf und/ oder ist datenleitend mit einer Speichervorrichtung verbunden/ verbindbar, auf der die Reinigungsinformationen gespeichert werden/ speicherbar sind. Die Reinigungsinformationen sind aus der Speichervorrichtung durch eine Auswerteeinrichtung abrufbar/ auslesbar, sodass anhand der Reinigungsinformationen durch die Auswerteeinrichtung ein Reinigungsprofil des Medizinprodukts bestimmbar ist. Die Datenverarbeitungseinrichtung ist eingerichtet, über den Temperaturverlauf bzw. die Temperaturkurve einen Reinigungsölvorgang zu erkennen und Rückschlüsse zu ziehen, ob der Reinigungsölvorgang geeignet durchgeführt wurde. Bevorzugt ist ein Drucksensor zur Bestimmung eines statischen und/ oder dynamischen Drucks einer Reinigungsflüssigkeit während des Reinigungsvorgangs vorgesehen.

Demnach ist es ermöglicht, dass der Reinigungsvorgang des Medizinprodukts mittels des Reinigungsprofils dokumentiert werden kann, ohne dass das Volumen und/oder das Gewicht des Medizinprodukts verändert werden würden. Es ist insbesondere auch vorgesehen, dass Verläufe der Sensordaten Bestandteil des Reinigungsprofils sein sollen. Die signalleitende Verbindung zwischen der Datenverarbeitungseinrichtung und dem Sensor kann beispielsweise mittels einer Kabelverbindung und/oder einer kabellosen Verbindung hergestellt sein. Neben der Bestimmung des Drucks des Reinigungsmittels und der Temperatur, ist es auch vorgesehen, dass beispielsweise eine Menge an Reinigungsöl, ein pH-Wert, eine Leitfähigkeit oder Ähnliches mittels eines oder mehrerer Sensoren bestimmt werden kann. Es ist vorgesehen, dass für alle ermittelten Daten auch Verläufe, Zyklen, Haltezeiten, Zeitverläufe oder Ähnliches erfasst werden können.

Es ist vorgesehen, dass die Datenverarbeitungseinrichtung beispielsweise auch ein sogenanntes SOC (System On a Chip) sein kann. Um die Datenverarbeitungseinrichtung an dem Halter, also innerhalb eines zu reinigenden und/ oder zu sterilisieren Bereiches, verwenden zu können, ist es vorgesehen, dass die Datenverarbeitungseinrichtung gegenüber Umwelteinflüssen geschützt ist.

Die Auswerteeinrichtung kann beispielsweise ein PC, ein Laptop, ein Tablett oder Ähnliches sein. Es ist darüber hinaus auch möglich, dass die Auswerteeinrichtung als ein Bestandteil eines weiteren Computersystems ausgebildet sein kann, beispielsweise eines Steuergeräts des Medizinprodukts.

Bei einer vorteilhaften Ausgestaltung der Erfindung ist vorgesehen, dass die Haltevorrichtung eine Zuleitung und zumindest eine von der Zuleitung abzweigende Reinigungsleitung, an die eine Spülaufnahme zur Aufnahme des Medizinprodukts insbesondere eines Handstücks angeschlossen ist, aufweist, wobei durch die Zuleitung, die Reinigungsleitung und die Spülaufnahme das Reinigungsmittel in zu reinigende Abschnitte insbesondere des Handstücks einleitbar ist. Handstücke im Sinne des Erfindungsgedankens meint die bereits zuvor beschriebenen Handstücke chirurgischer Instrumente.

Es ist vorteilhafterweise auch vorgesehen, dass die Haltevorrichtung eine Vielzahl von Reinigungsleitungen aufweisen kann, wobei jede Reinigungsleitung einer Spülaufnahme zugeordnet ist, sodass durch eine Überwachung der Reinigungsleitung ableitbar ist, ob ein Reinigungsvorgang entsprechend vorgegebener Parameter durchgeführt und die an den Spülaufnahmen angeordneten Medizinprodukte, insbesondere Handstücke adäquat gereinigt worden sind.

Eine vorteilhafte Ausgestaltung der Erfindung sieht vor, dass ein Temperatursensor und/ oder ein Drucksensor in der Zuleitung und/ oder der Reinigungsleitung und/ oder der Spülaufnahme angeordnet ist. Aufgrund einer thermodynamischen und hydraulischen Kupplung zwischen der Zuleitung, der Reinigungsleitungen und der Spülaufnahme ist es zur Ermittlung des Reinigungsprofils ausreichend, wenn jede Reinigungsleitung einen Drucksensor und die Zuleitung einen Temperatursensor aufweist.

Durch die ermittelte Temperatur in der Zuleitung und den Druck in den Reinigungsleitungen während des Reinigungsprozesses sind die Parameter definiert, mittels derer bestimmbar ist, dass der Reinigungsprozess von jedem Medizinprodukt insbesondere Handstück in einer Form durchlaufen wurde, die eine sachgemäße und hygienische Aufbereitung garantiert.

Um einen einfachen Aufbau der Haltevorrichtung zu erreichen, ist es auch möglich und vorgesehen, dass lediglich die Zuleitung sowohl einen Drucksensor, als auch einen Temperatursensor aufweisen kann. Dies ist insbesondere vorteilhaft, wenn hauptsächlich gleichartige Medizinprodukte insbesondere Handstücke mit ähnlichen Kontaminationsgraden gereinigt werden sollen.

Von besonderem Vorteil ist es, wenn ein Temperatursensor in der Haltevorrichtung integriert ist, welcher eingerichtet ist, (kontinuierlich) einen Temperaturverlauf bzw. eine Temperaturänderung bzw. eine Temperaturkurve (über die Zeit) zu erfassen und an die Datenverarbeitungseinrichtung weiterzugeben, und die Datenverarbeitungseinrichtung eingerichtet ist, einen Signalgeber anzusteuern, welcher eingerichtet ist, einem Anwender Hinweise bzw. Benachrichtigungen bzw. Informationen bzw. Warnungen zu geben, insbesondere über ein optisches und/oder akustisches Signal. Es ist demnach bevorzugt zumindest ein Temperatursensor vorgesehen, welcher einen Betrag und eine Dauer einer Temperaturänderung bzw. einen Temperaturverlauf über die Zeit erfasst.

Die Datenverarbeitungseinrichtung ist bevorzugt eingerichtet, über den Temperaturverlauf bzw. eine Temperaturänderung bzw. die Temperaturkurve (über die Zeit) einen Reinigungsprozess oder einen Reinigungsölvorgang zu erkennen bzw. Rückschlüsse zu ziehen, ob ein (vorgenommener) Reinigungsprozess oder ein (vorgenommener) Reinigungsölvorgang geeignet durchgeführt wurde.

Beispielsweise wird ein Reinigungsprozess erkannt, wenn die von dem Temperatursensor gemessene Temperatur eine vorbestimmte Temperatur, welche kennzeichnend für einen Reinigungsprozess ist, übersteigt. Die vorbestimmte Temperatur kann beispielsweise eine Temperatur zwischen 50° C und 70° C sein. Besonders bevorzugt ist die vorbestimmte Temperatur etwa 60°C.

Bevorzugt zeigt der Signalgeber dem Anwender an, wie lange der Reinigungsprozess oder der Reinigungsölvorgang durchzuführen sind. Alternativ oder zusätzlich zeigt der Signalgeber nach dem (durchgeführten) Reinigungsprozess oder nach dem (durchgeführten) Reinigungsölvorgang dem Anwender bevorzugt an, ob der entsprechende Reinigungsprozess oder Reinigungsölvorgang geeignet durchgeführt wurde. Alternativ oder zusätzlich gibt der Signalgeber dem Anwender bevorzugt einen Hinweis, welchen Schritt der Anwender als nächstes vornehmen muss.

Beispielsweise kann nach einem Erkennen des Reinigungsprozesses bzw. nach einem Durchführen des Reinigungsprozesses die Datenverarbeitungseinrichtung den Signalgeber, welcher bevorzugt ein optischer oder akustischer Signalgeber ist, ansteuern, dass dieser dem Anwender, bevorzugt durch ein optisches oder akustisches Signal, mitteilt, ob der Reinigungsprozess geeignet durchgeführt wurde.

Ferner kann nach einem Erkennen des Reinigungsprozesses bzw. nach einem Durchführen des Reinigungsprozesses die Datenverarbeitungseinrichtung den Signalgeber, welcher bevorzugt ein optischer oder akustischer Signalgeber ist, ansteuern, dass dieser den Anwender darauf hinweist, dass der Anwender in einem nächsten Schritt ein Pflegen des Medizinprodukts mit Reinigungsöl (Reinigungsölvorgang) vornehmen muss. Dies ist besonders wichtig, da der unbedingt notwendige Reinigungsölvorgang der Medizinprodukte nach dem Reinigungsprozess in einem Reinigungs- und Desinfektionsgerät (RDG) von dem Reinigungspersonal oft nicht gewusst oder vergessen wird. Beispielsweise können dem Anwender eine optische Warnmeldung (z.B. rote LED) und/oder ein geeignetes optisches Symbol angezeigt werden, welche den Anwender darauf hinweisen, dass der nächste vorzunehmende Schritt ein Pflegen des Medizinprodukts mit Reinigungsöl sein muss.

Die Datenverarbeitungseinrichtung ist bevorzugt eingerichtet, auch den Reinigungsölvorgang zu erkennen. Insbesondere sinkt nach einem Start des Reinigungsölvorgangs die Temperatur an dem Temperatursensor durch die Entspannungskälte des Treibmittels schlagartig (ca. 20K bzw. 20°C in der ersten Sekunde) ab. Da der Temperatursensor ständig/ kontinuierlich den Temperaturverlauf bzw. die Temperaturkurve aufzeichnet, erfasst er auch diesen schlagartigen Temperaturabfall innerhalb kürzester Zeit. Die Datenverarbeitungseinrichtung erkennt bevorzugt, ob der Reinigungsölvorgang durchgeführt wurde (ja/nein), und ob der Reinigungsölvorgang in geeigneter Weise durchgeführt wurde (insbesondere über die Form/ Art des Temperaturabfalls und dessen Dauer). Der Signalgeber kann dem Anwender somit bevorzugt auch mitteilen (durch ein optisches oder akustisches Signal), ob der Reinigungsölvorgang geeignet durchgeführt wurde. Darüber hinaus kann bevorzugt über die Form des Temperaturabfalls und dessen Dauer eine geeignete/ erforderliche Zeit des Reinigungsölvorgangs bestimmt und dem Anwender mitgeteilt werden (durch akustisches und/oder optisches Signal, beispielsweise durch eine erlöschende LED).

Ein bevorzugtes Ausführungsbeispiels ist dadurch gekennzeichnet, dass zwei Temperatursensoren, nämlich ein erster Temperatursensor und ein zweiter Temperatursensor vorgesehen sind, wobei der erste Temperatursensor in einer Zuleitung der Haltevorrichtung angebracht ist, und der zweite Temperatursensor außerhalb der Zuleitung an der Haltevorrichtung angebracht ist und für eine Referenzmessung vorgesehen ist. Es sind somit in anderen Worten besonders bevorzugt zwei Temperatursensoren vorgesehen.

Bei einer vorteilhaften Umsetzung des Erfindungsgedankens ist vorgesehen, dass die Datenverarbeitungseinrichtung mit einem Datenspeicher des Medizinprodukts verbunden/ verbindbar ist, wobei die Reinigungsinformationen in dem Datenspeicher gespeichert werden/ speicherbar sind. Demgemäß ist vorgesehen, dass die Datenverarbeitungseinrichtung die ermittelten Reinigungsinformationen in den Datenspeicher des Medizinprodukts schreibt. Bei Motoren und/oder Handstücken, die vor ihrer Verwendung an ein zugehöriges Steuergerät angeschlossen werden, ist es vorgesehen, dass die gespeicherten Reinigungsinformationen von dem Steuergerät ausgelesen und/oder zur Anzeige gebracht werden können. Eine derartige Datenübertragung kann beispielsweise über eine kabelgebundene Verbindung oder per Funk erfolgen.

Bei einer derartigen Ausgestaltung der Haltevorrichtung erfolgt eine Verknüpfung der Reinigungsinformationen mit einer Identität des Medizinprodukts dadurch, dass die dem Medizinprodukt zugeordneten Reinigungsinformationen direkt in den Datenspeicher des Medizinprodukts geschrieben werden.

Bei einer vorteilhaften Ausgestaltung der Haltevorrichtung ist vorgesehen, dass die Haltevorrichtung zumindest eine Identifikationsanordnung aufweist, die mit der Datenverarbeitungseinrichtung in Wirkverbindung gebracht/ bringbar ist und mittels derer das in der Haltevorrichtung angeordnete Medizinprodukt auf der Grundlage eines Identifikationsmerkmals identifizierbar ist, wobei mittels der Datenverarbeitungseinrichtung das Identifikationsmerkmal mit den Reinigungsinformationen zu Produktinformationen des Medizinprodukts verknüpfbar ist. Durch eine derartige Ausgestaltung der Erfindung wird es ermöglicht, dass die zu Produktinformationen verarbeiteten Reinigungsinformationen unabhängig von dem Medizinprodukt transferiert werden können und so geeignet sind auch im Rahmen einer externen Analyse, bei einer Bestandsüberwachung, bei der Überwachung von Serviceintervallen oder Ähnlichem verwendet zu werden.

Es ist vorgesehen, dass die Identifikationsanordnung beispielsweise ein Lesegerät zum Auslesen eines NFC-Chips sein kann, wobei das Identifikationsmerkmal ein an dem Medizinprodukt angeordneter NFC Chip ist. Identifikationsmerkmal im Sinne des Erfindungsgedankens meint alle Mittel sowie darin gespeicherte Identifikationsinformationen, die geeignet sind ein Objekt mittels eines automatisierbaren Verfahrens zu identifizieren. Bei der Verknüpfung der Reinigungsinformationen mit dem Identifikationsmerkmal werden die Reinigungsinformationen um die Identifikationsinformationen des Identifikationsmerkmals erweitert. Identifikationsmerkmals sind insbesondere NFC-Chips, Widerstandskodierungen, EEPROMs, Strichcodes, maschinenlesbare Zahlenfolgen oder Ähnliches. Es ist auch möglich, dass die Identifikationsanordnung teilweise oder vollständig in die Datenverarbeitungseinrichtung integriert ist.

Die Identifikationsanordnung kann sowohl mittels eines Kabels, als auch kabellos mit der Datenverarbeitungseinrichtung in Wirkverbindung gebracht sein. Es ist vorgesehen, dass die kabellose Verbindung beispielsweise mittels eines Funkstandards, beispielsweise per WLAN, hergestellt sein kann.

Eine vorteilhafte Ausgestaltung der Erfindung sieht vor, dass eine Identifikation des Medizinprodukts zumindest teilweise mittels eines drahtlosen Verfahrens erfolgt. Identifikation des Medizinprodukts im Sinne des Erfindungsgedankens meint insbesondere, welche Kombination aus Identifikationsanordnung und Identifikationsmerkmal verwendet wird, um das Medizinprodukt zu identifizieren. Drahtlose Verfahren umfassen dabei sowohl funkbasierte elektronische Verfahren, als auch beispielsweise optische Verfahren. Funkbasierte elektronische Verfahren sind beispielsweise die Verwendung von RFID-Technik, WLAN, NFC oder Ähnlichem. Optische Verfahren umfasst beispielsweise die Verwendung von QR-Codes, Strichcodes, maschinenlesbare Zahlenfolgen oder Ähnlichem.

Es ist auch möglich und erfindungsgemäß ggf. vorgesehen, dass eine Identifikation des Medizinprodukts zumindest teilweise mittels eines kabelgebundenen Verfahrens erfolgt. Bei den kabelgebundenen Verfahren ist es vorgesehen, dass zwischen der Identifikationsanordnung und dem Identifikationsmerkmal eine physische Verbindung, insbesondere eine Kabelverbindung, hergestellt wird/ist. Identifikationsmerkmale, die insbesondere für eine kabelgebundene Identifikation geeignet sind, sind beispielsweise Widerstandskodierungen, EEPROMs oder Ähnliche.

Es ist darüber hinaus möglich und ggf. vorgesehen, dass verschiedene Identifikationsmittel parallel genutzt werden können, um so eine Redundanz bei der Identifikation des Medizinprodukts zu erreichen. Hierdurch ist insbesondere eine Fehlerquote bei der Identifikation des Medizinprodukts verringert und eine Ausfallsicherheit erhöht.

Um eine direkte Übertragung der Reinigungsinformationen und/ oder Produktinformationen von der Datenverarbeitungseinrichtung zu der Auswerteeinrichtung zu erreichen, ist es erfindungsgemäß ggf. vorgesehen, dass die Datenverarbeitungseinrichtung eine Übertragungseinrichtung aufweist und/ oder mit einer Übertragungseinrichtung datenleitend in Wirkverbindung gebracht ist/ bringbar ist, wobei durch die Übertragungseinrichtung eine datenleitende Verbindung zwischen der Datenverarbeitungseinrichtung und der Auswerteeinrichtung hergestellt/ herstellbar ist mittels derer Reinigungsinformationen und/ oder Produktinformationen von der Datenverarbeitungseinrichtung an die Auswerteeinrichtung übertragbar sind. Eine derartige Ausgestaltung der erfindungsgemäßen Haltevorrichtung ist insbesondere vorteilhaft, wenn die zu reinigenden Medizinprodukte über keinen eigenen Datenspeicher verfügen. Derartige Medizinprodukte sind beispielsweise Drucklufthandstücke, wiederaufbereitbare Implantate, Werkzeuge, Instrumente oder Ähnliche. Es ist vorgesehen, dass die Auswerteeinrichtung beispielsweise ein PC, ein Tablett, ein Smartphone oder Ähnliches sein kann. Besonders vorteilhaft ist bei dieser Ausführungsform, dass beispielsweise der Inhalt eines Sterilcontainers überprüft werden kann, ohne dass dieser geöffnet und so seine Versiegelung gebrochen werden muss.

Weiter alternativ kann es vorgesehen sein, dass die Übertragungseinrichtung derart ausgestaltet ist, dass eine datenleitende Verbindung mit der Auswerteeinrichtung mittels eines Funkstandards, insbesondere eines Mobilfunkstandard, eines WLAN-Standard und/ oder eines Nahfeldkommunikationsstandards herstellbar ist. Durch die Verwendung eines Funkstandards ist die Anbindung der Auswerteeinrichtung an die Datenverarbeitungseinrichtung mittels der Übertragungseinrichtung vereinfacht, da auf standardisierte Protokolle zurückgegriffen werden kann. Es ist demgegenüber jedoch auch möglich, dass zum Herstellen der Verbindung ein generischer Funkstandard verwendet werden kann.

Die Erfindung betrifft auch ein Produktüberwachungssystem zur Überwachung medizinischer Produkte, dass zumindest eine der beschriebenen Haltevorrichtungen sowie zumindest eine Auswerteeinrichtung gemäß der Erfindung aufweist. Es ist auch möglich, dass das Produktüberwachungssystem eine Mehrzahl von Haltevorrichtungen aufweist, die mit einer oder mehreren Auswerteeinrichtungen in Wirkverbindung gebracht/in Wirkverbindung bringbar sind. Durch ein derartiges Produktüberwachungssystem ist es ermöglicht, dass eine Vielzahl von Medizinprodukten berührungslos mittels der Auswerteeinrichtung überwacht werden können. Gegenüber dem Stand der Technik ist insbesondere vorteilhaft, dass auch derartige Medizinprodukte individuell überwacht werden können, die andernfalls lediglich als Set überwachbar sind, beispielsweise wiederaufbereitbare Implantate.

Es ist vorgesehen, dass die Produktinformationen zwischen der Haltevorrichtung und der Auswerteeinrichtung sowohl direkt, als auch indirekt übertragbar sein können. Eine direkte Verbindung kann sowohl drahtlos, als auch kabelgebunden ausgebildet sein. Es ist vorgesehen, dass eine indirekte Verbindung insbesondere über den Datenspeicher des Medizinprodukts hergestellt sein kann, nämlich indem der Datenspeicher zunächst mit der Haltevorrichtung verbunden ist und die Produktinformationen auf dem Datenspeicher gespeichert werden und anschließend das Medizinprodukt mitsamt dem Datenspeicher an die Auswerteeinrichtung angeschlossen wird, sodass die Produktinformationen von der Auswerteeinrichtung aus dem Datenspeicher auslesbar sind.

Um eine Zuordnung der Produktinformationen zu dem korrekten Medizinprodukt durch die Auswerteeinrichtung zu ermöglichen, ist es vorgesehen, dass die Auswerteeinrichtung Mittel zum Auslesen von Informationsmerkmalen oder von in dem Datenspeicher des Medizinprodukts gespeicherten Produktinformationen aufweisen kann.

Eine vorteilhafte Ausgestaltung der Erfindung sieht vor, dass die Auswerteeinrichtung mit einem externen Speichermedium in Wirkverbindung gebracht ist/ bringbar ist, wobei die Reinigungsinformationen und/ oder Produktinformationen von der Datenverarbeitungseinrichtung über die Auswerteeinrichtung auf das externe Speichermedium übertragbar sind. Durch eine derartige Ausgestaltung des Produktüberwachungssystems wird es ermöglicht, dass Reinigungsinformation exportiert und auf externen Systemen ausgewertet und/oder gespeichert werden können. Hierdurch sind Maßnahmen zur Qualitätssicherung und hygienischen Überwachung in Bezug auf die Medizinprodukte erleichtert.

Bei einer vorteilhaften Ausgestaltung des erfindungsgemäßen Produktüberwachungssystems ist vorgesehen, dass das externe Speichermedium ein Cloudspeicher ist. Durch die Speicherung der Reinigungsinformationen in einem Cloudspeicher wird es ermöglicht, dass ein zuständiger Personenkreis, insbesondere Chirurgen, Personal der zentralen Sterilgutversorgungsabteilung, der Hersteller und Ähnliche, ortsunabhängig Informationen über einen Zustand der Medizinprodukte erhalten können. Darüber hinaus ist es dem Hersteller der medizinischen Produkte ermöglicht, dass Produktupdates, Rückrufaktionen oder Ähnliches zentralisiert und mit verringertem organisatorischen Aufwand durchgeführt und/ oder koordiniert werden können.

### Kurzbeschreibung der Figuren

Die Erfindung wird nachstehend anhand bevorzugter Ausführungsbeispiele unter Bezugnahme auf die begleitenden Figuren näher erläutert.
Fig. 1 zeigt eine isometrisch dargestellte Ansicht einer Ausführungsform der Haltevorrichtung,
Fig. 2 zeigt eine schematisch dargestellte und vereinfachte Schnittansicht der in Figur 1 gezeigten Haltevorrichtung,
Fig. 3 zeigt eine schematisch dargestellte Seitenansicht einer Ausführungsform der Haltevorrichtung und
Fig. 4 und 5 zeigen schematische Darstellungen von Ausführungsformen des Produktüberwachungssystems.

### Figurenbeschreibung

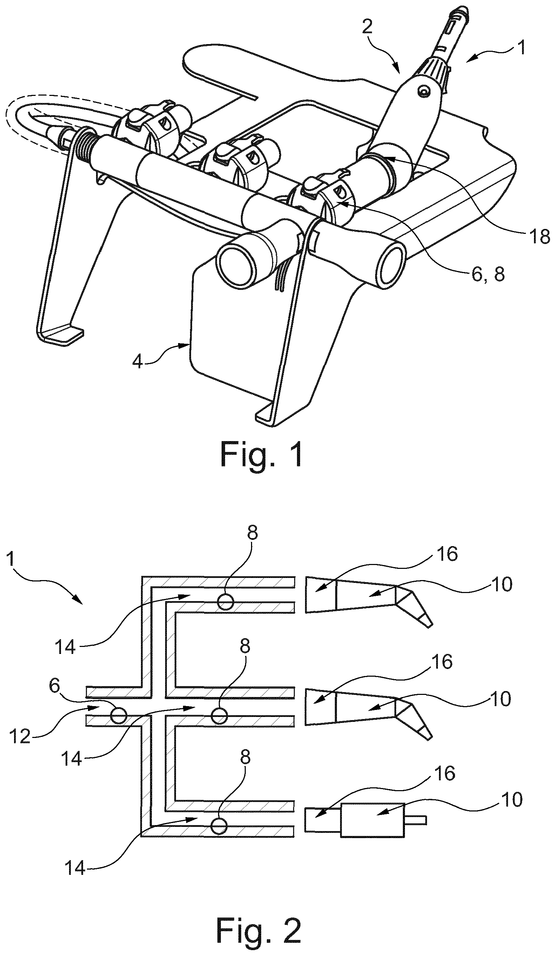

In den Figuren 1 bis 3 ist jeweils eine Ausführungsform einer Haltevorrichtung 1 zum Halten zumindest eines sterilisierbaren Medizinprodukts 2 während eines Reinigungsvorgangs sowie zum Lagern des Medizinprodukts 2 vor und nach dem Reinigungsvorgang gezeigt. Das Medizinprodukt 2 ist in der Haltevorrichtung 1 angeordnet und wird durch diese so gehalten, dass es seine Position weder während des Reinigungsvorgangs, noch während eines Transports verändern kann. Die dargestellten Haltevorrichtungen 1 sind derart ausgestaltet, dass sie bei Bedarf innerhalb eines nicht dargestellten Sterilcontainers angeordnet werden können.

Hierfür besteht die Haltevorrichtung 1 aus einem podest-artigen (Gitter) Rahmen, beispielsweise Blech-Rahmen, mit Stützfüßen 19, an welchem zumindest eine Rohrleitung 1b fixiert ist, die eine Anzahl von Abzweigungen 1c längs der Rohrleitung 1b aufweist, welche Anschlussstellen / Anschlussstutzen für die hohlförmigen Medizinprodukte 2 wie Handstücke bilden. Im Bereich dieser Anschlussstellen definiert der (Blech-) Rahmen jeweils eine Ausnehmung / Vertiefung oder Klemmbacken zur stabilen Lagerung des jeweiligen Medizinprodukts.

Die dargestellte Haltevorrichtung 1 weist jeweils eine Datenverarbeitungseinrichtung 4 sowie zumindest einen Temperatursensor 6 zur Erfassung einer Temperatur auf, der beispielsweise im Bereich der Anschlussstellen 1c angeordnet sein kann. Die in den Figuren 1 und 2 dargestellte Haltevorrichtung 1 weist darüber hinaus auch zumindest einen Drucksensor 8 zur Erfassung eines statischen Drucks einer Reinigungsflüssigkeit auf, der ebenfalls im Bereich der Anschlussstelle 1c angeordnet ist.

Die in Figur 2 gezeigte Schnittansicht, der in Figur 1 gezeigten Haltevorrichtung 1 zeigt den inneren Aufbau der Haltevorrichtung 1, die insbesondere an die Reinigung von Handstücken 10 angepasst ist. Die Haltevorrichtung 1 weist eine Zuleitung 12 und drei von der Zuleitung 12 abzweigende Reinigungsleitungen 14 auf, welche die vorstehend genannten Anschlussstellen 1c bilden. An die Reinigungsleitungen 14 ist jeweils eine Spülaufnahme 16 zur (fluidischen) Aufnahme/Anschluss eines Handstücks 10 angeschlossen/ausgebildet. Durch die Zuleitung 12, die Reinigungsleitungen 14 und die Spülaufnahme 16 ist das Reinigungsmittel in die Handstücke 10 einleitbar. In jeder der Reinigungsleitungen 14 ist jeweils ein Drucksensor 8 angeordnet und in der Zuleitung 12 ist ein Temperatursensor 6 angeordnet. Neben dem Temperatursensor 6 kann an der Haltevorrichtung 1 außerhalb der Zuleitung 12 noch ein zweiter, nicht gezeigter, Temperatursensor angebracht sein, welcher einer Referenzmessung dient. Die datenleitende Verbindung zwischen den Sensoren 6,8 und der Datenverarbeitungseinrichtung 4 ist in Figur 2 nicht gezeigt.

Die Datenverarbeitungseinrichtung 4 ist signalleitend mit den Sensoren 6,8 verbunden und verarbeitet die Sensorsignale zu Reinigungsinformationen.

Der vorgesehene Temperatursensor 6 erfasst kontinuierlich einen Temperaturverlauf und gibt diesen an die Datenverarbeitungseinrichtung 4 weiter. Beispielsweise erkennt die Datenverarbeitungseinrichtung 4, dass ein Reinigungsprozess in einem Reinigungs- und Desinfektionsgerät vorgenommen wird, wenn die Temperatur einen vorbestimmten Wert, welcher etwa bei 60°C liegt, überschreitet. Die Datenverarbeitungseinrichtung 4 erkennt ferner, ob der Reinigungsprozess in geeigneter Weise durchgeführt wurde, und kann einen (nicht gezeigten) Signalgeber, etwa eine LED, ansteuern, so dass der Signalgeber einem Anwender (akustisch oder optisch) anzeigt, dass der Reinigungsprozess in geeigneter Weise durchgeführt wurde.

Weiterhin kann die Datenverarbeitungseinrichtung 4 den Signalgeber ansteuern, dass der Signalgeber den Anwender darauf hinweist, dass in einem nächsten Schritt (nach dem Reinigungsprozess in dem Reinigungs- und Desinfektionsgerät) ein Pflegen des Medizinprodukts mit Reinigungsöl (Reinigungsölvorgang) erfolgen muss, insbesondere da dieser Schritt von einem Reinigungspersonal häufig nicht gewusst oder vergessen wird.

Nach einem Start des Reinigungsölvorgangs sinkt die Temperatur an dem Temperatursensor 6 durch Entspannungskälte des Treibmittels schlagartig (etwa 20K/ 20°C in der ersten Sekunde) ab. Dieser Temperaturabfall wird von dem Temperatursensor 6 erfasst und von der Datenverarbeitungseinrichtung 4 erkannt. Somit erkennt die Datenverarbeitungseinrichtung 4 auch, ob der Reinigungsölvorgang durchgeführt wird. Über eine Form und eine Dauer des Temperaturabfalls erkennt die Datenverarbeitungseinrichtung 4 darüber hinaus auch, ob der Reinigungsölvorgang geeignet durchgeführt wurde bzw. kann die Datenverarbeitungseinrichtung 4 dem Anwender über den Signalgeber eine geeignete/ erforderliche Zeit des Reinigungsölvorgangs mitteilen.

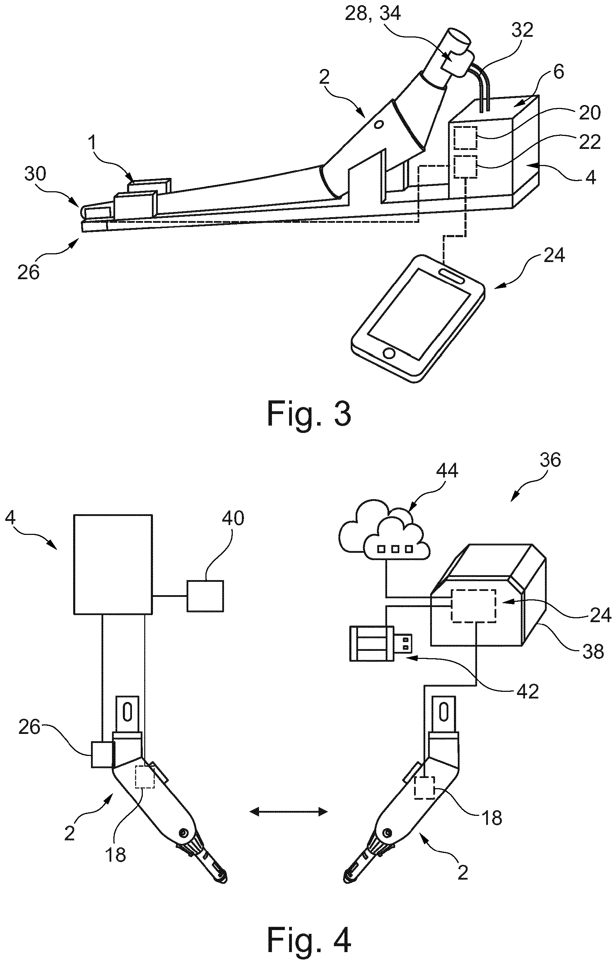

Die Datenverarbeitungseinrichtung 4 der in Figur 1 dargestellten Haltevorrichtung 1 ist mit einem Datenspeicher 18 des (im) Medizinprodukts 2 verbunden, auf der die Reinigungsinformationen gespeichert werden. Demgegenüber weist die in Figur 3 dargestellte Haltevorrichtung 1 eine interne Speichervorrichtung 20 (alternativ zum externen Datenspeicher 18) auf.

Die Datenverarbeitungseinrichtung 4 der in Figur 3 dargestellten Haltevorrichtung 1 weist eine Übertragungseinrichtung 22 auf. Die Übertragungseinrichtung 22 ist mittels eines Funkstandards datenleitend mit einer (vorzugsweise) als Smartphone ausgebildeten Auswerteeinrichtung 24 verbunden. Darüber hinaus weist die Haltevorrichtung 1 auch eine erste Identifikationsanordnung 26 und eine zweite Identifikationsanordnung 28 auf, die beide mit der Datenverarbeitungseinrichtung 4 verbunden sind. Die erste Identifikationsanordnung 26 ist an ein erstes Identifikationsmerkmal 30 des Medizinprodukts 2 z. B. einen Effektor Werkzeugschaft angepasst und identifiziert dieses vorzugsweise drahtlos. Die Anbindung der ersten Identifikationsanordnung 26 an die Datenverarbeitungseinrichtung 4 ist ebenfalls bevorzugt als eine drahtlose Verbindung ausgebildet. Die zweite Identifikationsanordnung 28 ist bevorzugt mittels einer Drahtverbindung 32 mit der Datenverarbeitungseinrichtung 4 verbunden. Sie ist darüber hinaus mit einem zweiten Identifikationsmerkmal 34 des Medizinprodukts 2 z. B. einem Anschlussabschnitt des Medizinprodukts kontaktiert. Durch die Identifikationsanordnungen 26,28 ist anhand von in den Identifikationsmerkmalen 30,34 gespeicherten Identifikationsinformationen eine Identität des Medizinprodukts 2 bestimmbar. Mittels der Datenverarbeitungseinrichtung 4 werden die Identifikationsinformationen der Identifikationsmerkmale 30,34 (Abtaster, Scanner etc.) mit den Reinigungsinformationen zu Produktinformationen des Medizinprodukts 2 verknüpft.

Über die datenleitende Verbindung zwischen der Auswerteeinrichtung 24 und der Datenverarbeitungseinrichtung 4 werden sowohl die momentan verfügbaren Produktinformationen, als auch die in der Speichervorrichtung 20 vorhandenen Produktinformationen an die Auswerteeinrichtung 24 übertragen.

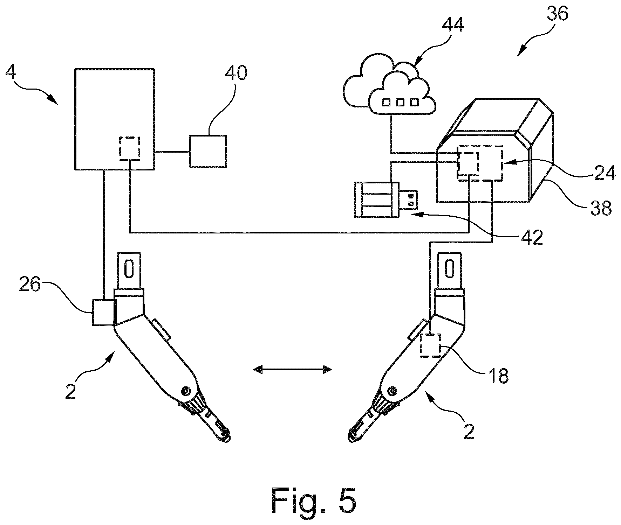

In den Figuren 4 und 5 sind jeweils schematisch dargestellte Ausführungsformen eines Produktüberwachungssystems 36 gezeigt, das eine (separate) Auswerteeinrichtung 24 und eine Haltevorrichtung 1 mit einer Datenverarbeitungseinrichtung 4 umfasst. Die Auswerteeinrichtung 24 ist dabei beispielsweise in ein Steuergerät 38 zur Steuerung eines motorbetriebenen Medizinprodukts 2 integriert, in welchem der Datenspeicher 18 untergebracht ist. Die Haltevorrichtung 1 weist einen Energiespeicher 40 auf, der die daran angeordnete Datenverarbeitungseinrichtung 4 mit elektrischer Energie versorgt und so ihren autonomen Betrieb ermöglicht. Darüber hinaus weist die Haltevorrichtung 1 des dargestellten Produktüberwachungssystems 36 jeweils eine erste Identifikationsanordnung 26 auf, sodass Medizinprodukte 2 mit Identifikationsmerkmalen identifizierbar sind. Die Auswerteeinrichtung 24 ist bei den dargestellten Ausführungsformen gemäß der Fig. 4 und 5 bevorzugt mit einem USB-Stick 42 und mit einem Cloudspeicher 44 verbunden. Die Produktinformationen können somit von der Datenverarbeitungseinrichtung 4 der Haltevorrichtung 1 über die (externe) Auswerteeinrichtung 24 sowohl auf den USB-Stick 42, als auch in den Cloudspeicher 44 übertragen werden.

Bei dem in Figur 4 dargestellten Produktüberwachungssystem 36 weist die Datenverarbeitungseinrichtung 4. Keine zusätzliche Übertragungseinrichtung 22 auf. Die Verbindung zwischen der Datenverarbeitungseinrichtung 4 und der Auswerteeinrichtung 24 erfolgt in diesem Fall indirekt, mittels des Datenspeichers 18 des Medizinprodukt 2. Demgegenüber weisen bei dem in Figur 5 dargestellten Produktüberwachungssystem 36 sowohl die Datenverarbeitungseinrichtung 4, als auch die Auswerteeinrichtung 24 eine Übertragungseinrichtung 22 auf, sodass bei der dargestellten Ausführungsform auch eine direkte Verbindung zwischen der Datenverarbeitungseinrichtung 4 und der Auswerteeinrichtung 24 hergestellt ist. Die Identifikation des Medizinprodukts 2 erfolgt auf der Grundlage von im Datenspeicher 18 hinterlegten Daten, sodass die Produktinformationen durch die Auswerteeinrichtung 24 dem aktuell angeschlossenen Medizinprodukt 2 zugeordnet werden können.

### Bezugszeichenliste

- 1.: Haltevorrichtung
- 2.: Medizinprodukt
- 4.: Datenverarbeitungseinrichtung
- 6.: Temperatursensor
- 8.: Drucksensor
- 10.: Handstück
- 12.: Zuleitung
- 14.: Reinigungsleitungen
- 16.: Spülaufnahme
- 18.: Datenspeicher
- 20.: Speichervorrichtung
- 22.: Übertragungseinrichtung
- 24.: Auswerteeinrichtung
- 26.: erste Identifikationsanordnung
- 28.: zweite Identifikationsanordnung
- 30.: erstes Identifikationsmerkmal
- 32.: Drahtverbindung
- 34.: zweites Identifikationsmerkmal
- 36.: Produktüberwachungssystem
- 38.: Steuergerät
- 40.: Energiespeicher
- 42.: USB-Stick
- 44.: Cloudspeicher

## Patentansprüche

1. Haltevorrichtung (1) zur Verwendung innerhalb eines zu reinigenden und innerhalb eines zu sterilisierenden Bereichs, zum Halten zumindest eines sterilisierbaren Medizinprodukts (2) während eines Reinigungsvorgangs sowie zum Lagern des Medizinprodukts (2) vor und nach dem Reinigungsvorgang, wobei
- die Haltevorrichtung (1) eine Datenverarbeitungseinrichtung (4) aufweist, die mit zumindest einem Temperatursensor (6) zur Bestimmung einer Temperatur während des Reinigungsvorgangs signalleitend verbunden ist, wobei der Temperatursensor (6) eingerichtet ist, einen Temperaturverlauf bzw. eine Temperaturkurve zu erfassen und an die Datenverarbeitungseinrichtung (4) weiterzugeben,
- die Datenverarbeitungseinrichtung (4) die Sensorsignale zu Reinigungsinformationen verarbeitet,
- die Datenverarbeitungseinrichtung (4) eine Speichervorrichtung (20) aufweist und/ oder datenleitend mit einer Speichervorrichtung (20) verbunden/ verbindbar ist, auf der die Reinigungsinformationen gespeichert werden/ speicherbar sind und
- die Reinigungsinformationen von der Speichervorrichtung (20) durch eine Auswerteeinrichtung (24) abrufbar/ auslesbar sind, sodass anhand der Reinigungsinformationen durch die Auswerteeinrichtung (24) ein Reinigungsprofil des Medizinprodukts (2) bestimmbar ist,
**dadurch gekennzeichnet, dass**
die Datenverarbeitungseinrichtung (4) eingerichtet ist, über den Temperaturverlauf bzw. die Temperaturkurve einen Reinigungsölvorgang zu erkennen und Rückschlüsse zu ziehen, ob der Reinigungsölvorgang geeignet durchgeführt wurde.

2. Haltevorrichtung (1) nach Anspruch 1, **dadurch gekennzeichnet, dass** die Haltevorrichtung (1) eine Zuleitung (12) und eine Vielzahl von von der Zuleitung (12) abzweigende Reinigungsleitungen (14), an welchen jeweils eine Spülaufnahme (16) zur Aufnahme eines Handstücks (10) angeschlossen ist, aufweist, wobei durch die Zuleitung (12), die Reinigungsleitung (14) und die Spülaufnahme (16) das Reinigungsmittel in zu reinigende Abschnitte des Handstücks (10) einleitbar ist.

3. Haltevorrichtung (1) nach Anspruch 2, **dadurch gekennzeichnet, dass** der Temperatursensor (6) in der Zuleitung (12) und/ oder der Reinigungsleitung (14) und/ oder der Spülaufnahme (16) angeordnet ist.

4. Haltevorrichtung (1) nach einem der Ansprüche 1 bis 3, **dadurch gekennzeichnet, dass** die Datenverarbeitungseinrichtung (4) eingerichtet ist, auf der Grundlage eines schlagartigen Absinkens der von dem Temperatursensor (6) erfassten Temperatur zu erkennen, dass der Reinigungsölvorgang durchgeführt wird.

5. Haltevorrichtung (1) nach einem der Ansprüche 1 bis 4, **dadurch gekennzeichnet, dass** die Datenverarbeitungseinrichtung (4) eingerichtet ist, einen Signalgeber anzusteuern, welcher eingerichtet ist, einem Anwender Hinweise zu geben, insbesondere über ein optisches und/oder akustisches Signal, und der Signalgeber dem Anwender anzeigt, wie lange der Reinigungsölvorgang durchzuführen ist, und/oder nach dem durchgeführten Reinigungsölvorgang dem Anwender anzeigt, ob der entsprechende Reinigungsölvorgang geeignet durchgeführt wurde und/oder dem Anwender einen Hinweis gibt, welchen Schritt der Anwender als nächstes vornehmen muss.

6. Haltevorrichtung (1) nach einem der Ansprüche 1 bis 5, **dadurch gekennzeichnet, dass** zwei Temperatursensoren, nämlich ein erster Temperatursensor (6) und ein zweiter Temperatursensor vorgesehen sind, wobei der erste Temperatursensor (6) in einer Zuleitung (12) der Haltevorrichtung (1) angebracht ist, und der zweite Temperatursensor außerhalb der Zuleitung (12) an der Haltevorrichtung (1) angebracht ist und für eine Referenzmessung vorgesehen ist.

7. Haltevorrichtung (1) nach einem der Ansprüche 1 bis 6, **dadurch gekennzeichnet, dass** die Datenverarbeitungseinrichtung (4) mit einem Datenspeicher (18) des Medizinprodukts (2) verbunden/ verbindbar ist, wobei die Reinigungsinformationen in dem Datenspeicher (18) gespeichert werden/ speicherbar sind.

8. Haltevorrichtung (1) nach einem der Ansprüche 1 bis 7, **dadurch gekennzeichnet, dass** die Haltevorrichtung (1) zumindest eine Identifikationsanordnung (26,28) aufweist, die mit der Datenverarbeitungseinrichtung (4) in Wirkverbindung gebracht! bringbar ist und mittels derer das in der Haltevorrichtung (1) angeordnete Medizinprodukt (2) auf der Grundlage eines Identifikationsmerkmals (30,34) identifizierbar ist, wobei mittels der Datenverarbeitungseinrichtung (4) das Identifikationsmerkmal (30,34) mit den Reinigungsinformationen zu Produktinformationen des Medizinprodukts (2) verknüpfbar ist.

9. Haltevorrichtung (1) nach einem der Ansprüche 1 bis 8, **dadurch gekennzeichnet, dass** die Datenverarbeitungseinrichtung (4) eine Übertragungseinrichtung (22) aufweist und/ oder mit einer Übertragungseinrichtung (22) datenleitend in Wirkverbindung gebracht ist/ bringbar ist, wobei durch die Übertragungseinrichtung (22) eine datenleitende Verbindung zwischen der Datenverarbeitungseinrichtung (4) und der Auswerteeinrichtung (24) hergestellt/ herstellbar ist mittels derer Reinigungsinformationen und/ oder Produktinformationen von der Datenverarbeitungseinrichtung (4) an die Auswerteeinrichtung (24) übertragbar sind.

10. Haltevorrichtung (1) nach einem der Ansprüche 1 bis 9, **dadurch gekennzeichnet, dass** die Haltevorrichtung (1) ferner einen Drucksensor (8) zur Bestimmung eines statischen und/oder dynamischen Drucks eines Reinigungsmittels aufweist.

11. Haltevorrichtung (1) nach einem der Ansprüche 1 bis 10, **dadurch gekennzeichnet, dass** die Haltevorrichtung (1) einen podest-artigen Rahmen mit Stützfüßen (19) aufweist.

12. Haltevorrichtung (1) nach einem der Ansprüche 1 bis 11, **dadurch gekennzeichnet, dass** die Haltevorrichtung einen Energiespeicher (40) aufweist, welcher die Datenverarbeitungseinrichtung (4) mit elektrischer Energie versorgt.

13. Produktüberwachungssystem (36) zur Überwachung Medizinischer Produkte mit zumindest einer Haltevorrichtung (1) nach einem der Ansprüche 1 bis 12 und zumindest einer Auswerteeinrichtung (24), wobei die Auswerteeinrichtung (24) mit einem externen Speichermedium (34, 36), welches bevorzugt ein Cloudspeicher (44) ist, in Wirkverbindung gebracht ist/ bringbar ist, wobei die Reinigungsinformationen und/ oder Produktinformationen von der Datenverarbeitungseinrichtung (4) über die Auswerteeinrichtung (24) auf das externe Speichermedium (34, 36) übertragbar sind.

## Claims

1. A holding apparatus (1) for use within an area to be cleaned and within an area to be sterilized, for holding at least one sterilizable medical product (2) during a cleaning process as well as for storing the medical product (2) before and after the cleaning process, wherein
- the holding apparatus (1) has a data processing device (4) which is connected in a signal-conducting manner to at least one temperature sensor (6) for determining a temperature during the cleaning process, wherein the temperature sensor (6) is configured to detect a temperature profile or a temperature curve and to transmit it to the data processing device (4),
- the data processing device (4) processes the sensor signals to cleaning information,
- the data processing device (4) has a storage device (20) and/or is/can be connected in a data-conducting manner to a storage device (20) on which the cleaning information is/can be stored, and
- the cleaning information can be retrieved/read out from the storage device (20) by an evaluation device (24), so that a cleaning profile of the medical product (2) can be determined by the evaluation device (24) based on the cleaning information,
**characterized in that**
the data processing device (4) is configured to recognize a cleaning oil process via the temperature profile or the temperature curve and to draw conclusions as to whether the cleaning oil process has been carried out appropriately.

2. The holding apparatus (1) according to claim 1, **characterized in that** the holding apparatus (1) has a supply line (12) and a plurality of cleaning lines (14) which branch off from the supply line (12) and to which a respective rinsing receptacle (16) for receiving a handpiece (10) is connected, wherein the cleaning agent can be supplied through the supply line (12), the cleaning line (14) and the rinsing receptacle (16) to sections of the handpiece (10) to be cleaned.

3. The holding apparatus (1) according to claim 2, **characterized in that** the temperature sensor (6) is arranged in the supply line (12) and/or in the cleaning line (14) and/or in the rinsing receptacle (16).

4. The holding device (1) according to one of claims 1 to 3, **characterized in that** the data processing device (4) is configured to detect that the cleaning oil process is being carried out on the basis of a sudden drop in the temperature detected by the temperature sensor (6).

5. The holding apparatus (1) according to one of claims 1 to 4, **characterized in that** the data processing device (4) is configured to control a signal generator which is configured to give instructions to a user, in particular via an optical and/or acoustic signal, and the signal generator indicates to the user how long the cleaning oil process has to be performed, and/or after the cleaning oil process has been performed, indicates to the user whether the corresponding cleaning oil process has been performed suitably and/or indicates to the user which step the user has to perform next.

6. The holding apparatus (1) according to one of claims 1 to 5, **characterized in that** two temperature sensors, i.e. a first temperature sensor (6) and a second temperature sensor, are provided, wherein the first temperature sensor (6) is provided in a supply line (12) of the holding apparatus (1), and the second temperature sensor is provided outside the supply line (12) on the holding apparatus (1) and is provided for a reference measurement.

7. The holding apparatus (1) according to one of claims 1 to 6, **characterized in that** the data processing device (4) is/can be connected to a data memory (18) of the medical product (2), wherein the cleaning information is/can be stored in the data memory (18).

8. The holding apparatus (1) according to one of claims 1 to 7, **characterized in that** the holding apparatus (1) has at least one identification assembly (26, 28) which is/can be brought into operative connection with the data processing device (4) and by means of which the medical product (2) arranged in the holding apparatus (1) can be identified on the basis of an identification feature (30, 34), wherein by means of the data processing device (4) the identification feature (30, 34) can be linked with the cleaning information to become product information of the medical product (2).

9. The holding apparatus (1) according to one of claims 1 to 8, **characterized in that** the data processing device (4) has a transmission device (22) and/or is/can be brought into operative connection with a transmission device (22) in a data-conducting manner, wherein a data-conducting connection between the data processing device (4) and the evaluation device (24) is/can be established via the transmission device (22), by means of which cleaning information and/or product information can be transmitted from the data processing device (4) to the evaluation device (24).

10. The holding device (1) according to one of claims 1 to 9, **characterized in that** the holding device (1) further comprises a pressure sensor (8) for determining a static and/or dynamic pressure of a cleaning agent.

11. The holding device (1) according to one of claims 1 to 10, **characterized in that** the holding device (1) has a platform-like frame with supporting feet (19).

12. The holding device (1) according to one of claims 1 to 11, **characterized in that** the holding device has an energy storage device (40) which supplies the data processing device (4) with electrical energy.

13. A product monitoring system (36) for monitoring medical products with at least one holding apparatus (1) according to one of claims 1 to 12 and at least one evaluation device (24), wherein the evaluation device (24) is/can be brought into operative connection with an external storage medium (34, 36), which is preferably a cloud storage (44), wherein the cleaning information and/or product information can be transferred from the data processing device (4) via the evaluation device (24) to the external storage medium (34, 36).

## Revendications

1. Dispositif de retenue (1) pour une utilisation à l'intérieur d'une zone à nettoyer et à l'intérieur d'une zone à stériliser, pour retenir au moins un produit médical stérilisable (2) pendant un processus de nettoyage, ainsi que pour stocker le produit médical (2) avant et après le processus de nettoyage, dans lequel
- le dispositif de retenue (1) présente un appareil de traitement de données (4) qui est relié par conduction de signal à au moins un capteur de température (6) pour la détermination d'une température pendant le processus de nettoyage, dans lequel le capteur de température (6) est conçu pour détecter une évolution des températures ou une courbe des températures et pour la transmettre à l'appareil de traitement de données (4),
- l'appareil de traitement de données (4) transforme les signaux de capteur en informations relatives au nettoyage,
- l'appareil de traitement de données (4) présente un dispositif de stockage (20) et/ou est/peut être relié par conduction de données au dispositif de stockage (20), sur lequel les informations relatives au nettoyage sont stockées/peuvent être stockées et
- les informations relatives au nettoyage peuvent être consultées/peuvent être lues depuis le dispositif de stockage (20) par un appareil d'évaluation (24), de sorte qu'un profil de nettoyage du produit médical (2) puisse être déterminé par l'appareil d'évaluation (24) à l'aide des informations relatives au nettoyage, **caractérisé en ce que**
l'appareil de traitement de données (4) est conçu pour détecter un processus d'huile de nettoyage par le biais de l'évolution des températures ou de la courbe des températures et pour conclure si le processus d'huile de nettoyage a été réalisée de manière appropriée.

2. Dispositif de retenue (1) selon la revendication 1, **caractérisé en ce que** le dispositif de retenue (1) présente une ligne d'alimentation (12) et une pluralité de conduites de nettoyage (14) partant de la ligne d'alimentation (12), conduites auxquelles respectivement est raccordé un logement de rinçage (16) pour recevoir une poignée (10), dans lequel le produit de nettoyage peut être introduit dans la section à nettoyer de la poignée (10) par la ligne d'alimentation (12), la conduite de nettoyage (14) et le logement de rinçage (16).

3. Dispositif de retenue (1) selon la revendication 2, **caractérisé en ce que** le capteur de température (6) est disposé dans la ligne d'alimentation (12) et/ou la conduite de nettoyage (14) et/ou le logement de rinçage (16).

4. Dispositif de retenue (1) selon l'une quelconque des revendications 1 à 3, **caractérisé en ce que** l'appareil de traitement de données (4) est conçu pour détecter, sur la base d'une chute brutale de la température détectée par le capteur de température (6), si le processus d'huile de nettoyage est réalisé.

5. Dispositif de retenue (1) selon l'une quelconque des revendications 1 à 4, **caractérisé en ce que** l'appareil de traitement de données (4) est conçu pour commander un émetteur de signal qui est conçu pour donner des indications à un utilisateur, en particulier par le biais d'un signal optique et/ou acoustique, et l'émetteur de signal indique à l'utilisateur pendant combien de temps le processus d'huile de nettoyage doit être réalisé, et/ou indique à l'utilisateur après le processus d'huile de nettoyage réalisé si le processus d'huile de nettoyage correspondant a été réalisé de manière appropriée et/ou donne une indication à l'utilisateur sur la prochaine étape que l'utilisateur doit entreprendre.

6. Dispositif de retenue (1) selon l'une quelconque des revendications 1 à 5, **caractérisé en ce que** deux capteurs de température, à savoir un premier capteur de température (6) et un second capteur de température sont prévus, dans lequel le premier capteur de température (6) est monté dans une ligne d'alimentation (12) du dispositif de retenue (1), et le second capteur de température est monté à l'extérieur de la ligne d'alimentation (12) au niveau du dispositif de retenue (1) et est prévu pour une mesure de référence.

7. Dispositif de retenue (1) selon l'une quelconque des revendications 1 à 6, **caractérisé en ce que** l'appareil de traitement de données (4) est/peut être relié à une mémoire de données (18) du produit médical (2), dans lequel les informations relatives au nettoyage sont/peuvent être stockées dans la mémoire de données (18).

8. Dispositif de retenue (1) selon l'une quelconque des revendications 1 à 7, **caractérisé en ce que** le dispositif de retenue (1) présente au moins un agencement d'identification (26, 28) qui est/peut être amené en liaison fonctionnelle avec l'appareil de traitement de données (4) et au moyen duquel le produit médical (2) disposé dans le dispositif de retenue (1) peut être identifié sur la base d'une caractéristique d'identification (30, 34), dans lequel la caractéristique d'identification (30, 34) peut être associée au moyen de l'appareil de traitement de données (4) aux informations de nettoyage pour obtenir des informations sur le produit du produit médical (2).

9. Dispositif de retenue (1) selon l'une quelconque des revendications 1 à 8, **caractérisé en ce que** l'appareil de traitement de données (4) présente un appareil de transmission (22) et/ou est/peut être amené en liaison fonctionnelle par conduction de données avec un appareil de transmission (22), dans lequel une liaison par conduction de données est/peut être établie par l'appareil de transmission (22) entre l'appareil de traitement de données (4) et l'appareil d'évaluation (24) au moyen de laquelle des informations relatives au nettoyage et/ou des informations relatives au produit peuvent être transmises de l'appareil de traitement de données (4) à l'appareil d'évaluation (24).

10. Dispositif de retenue (1) selon l'une quelconque des revendications 1 à 9, **caractérisé en ce que** le dispositif de retenue (1) présente en outre un capteur de pression (8) pour la détermination d'une pression statique et/ou dynamique d'un produit de nettoyage.

11. Dispositif de retenue (1) selon l'une quelconque des revendications 1 à 10, **caractérisé en ce que** le dispositif de retenue (1) présente un cadre en forme de piédestal avec pieds d'appui (19).

12. Dispositif de retenue (1) selon l'une quelconque des revendications 1 à 11, **caractérisé en ce que** le dispositif de retenue présente un dispositif de stockage d'énergie (40) qui alimente l'appareil de traitement de données (4) en énergie électrique.

13. Système de surveillance de produit (36) pour la surveillance de produits médicaux avec au moins un dispositif de retenue (1) selon l'une quelconque des revendications 1 à 12 et au moins un appareil d'évaluation (24), dans lequel l'appareil d'évaluation (24) est/peut être amené en liaison fonctionnelle avec un support de stockage externe (34, 36) qui est de préférence un dispositif de stockage dans le cloud (44), dans lequel les informations relatives au nettoyage et/ou les informations relatives au produit peuvent être transmises de l'appareil de traitement de données (4) au support de stockage externe (34, 36) par le biais de l'appareil d'évaluation (24).
